# EUROPÄISCHE PATENTANMELDUNG

(11) **EP 0 602 653 A2**
(43) Veröffentlichungstag der Anmeldung: **22.06.1994**
(21) Anmeldenummer: 93120339.2
(22) Anmeldetag: 16.12.1993
(51) Int. Cl.: C07D 491/048, C07B 53/00, C07B 35/02

(54) **Asymmetrische Hydrierung von Dihydrofuroimidazol-derivaten**

(30) Priorität: 18.12.1992 CH 3873/92; 18.02.1993 CH 498/93
(71) Anmelder: LONZA AG, CH-4002 Basel (CH)
(72) Erfinder: Dr. Eyer, Martin, Brig-Glis Kanton Wallis (CH); Dr. Fuchs, Rudolf, Sion (Kanton Wallis) (CH); Dr. McGarrity, John, Visp (Kanton Wallis) (CH)
(74) Vertreter: Weinhold, Peter, Dr.

(57) **Zusammenfassung**

Es wird ein neues Verfahren zur asymmetrischen Hydrierung von Furoimidazolderivaten der allgemeinen Formel 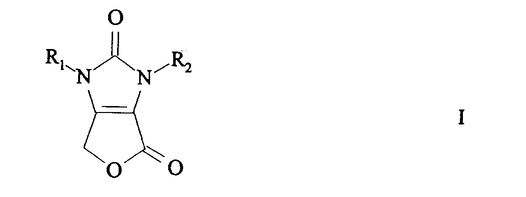 mit Wasserstoff in Gegenwart eines Homogenkatalysators zu den entsprechenden diastereomeren Tetrahydrofuroimidazolderivaten der allgemeinen Formel 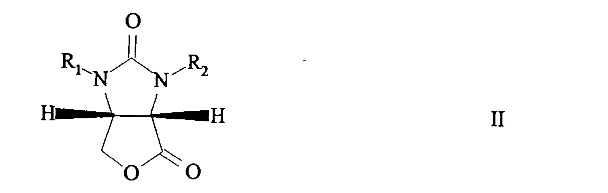 beschrieben.

Die Tetrahydrofuroimidazolderivate der allgemeinen Formel II sind Zwischenprodukte zur Herstellung des Vitamins (+)-Biotin.

## Beschreibung

Die Erfindung betrifft ein neues Verfahren zur asymmetrischen Hydrierung von Dihydrofuroimidazolderivaten der allgemeinen Formel 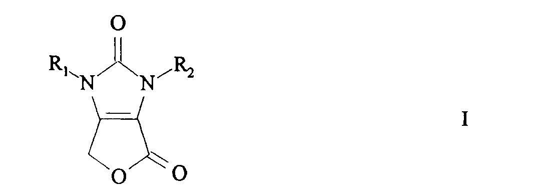 worin R₁ eine auf bekannte Weise abspaltbare Schutzgruppe bedeutet und R₂ für Wasserstoff oder eine auf bekannte Weise abspaltbare Schutzgruppe steht,mit Wasserstoff in Gegenwart eines Homogenkatalysators zu den entsprechenden diastereomeren Tetrahydrofuroimidazolderivaten der allgemeinen Formel 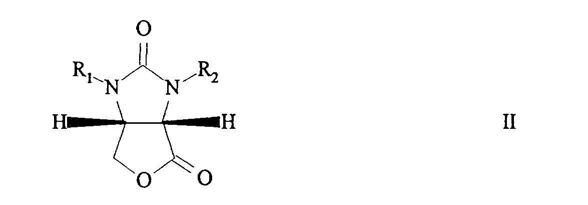 worin R₁ und R₂ die genannte Bedeutung haben.

Die Tetrahydrofuroimidazole der allgemeinen Formel II sind wichtige Zwischenprodukte in der Synthese von (+) Biotin, einem für den Menschen essentiellen Vitamin, das auch als Vitamin H bezeichnet wird. (+) Biotin wird ausserdem als Pharmazeutika zur Behandlung von Dermatose oder als Futtermittelzusatz mit wachstumssteigernder Wirkung für Nutztiere eingesetzt.

Die meisten bekannten (+) Biotin-Synthesen verfolgen das Ziel,geeignete Vorstufen über zum Teil sehr aufwendige Racematspaltungsmethoden mit zum Teil sehr teuren Spaltungsagentien zu trennen und mit dem resultierenden Diastereomeren die (+) Biotinsynthese weiterzuverfolgen (vgl. z. B. DE-PS 2 058 248). Gemäss EP-PS 273 270 erreichte man dann erstmals die Einführung der relevanten optisch aktiven Zentren, das heisst die 3aS- und 6aR-Position der Biotinringstruktur, über eine asymmetrische Hydrierung entsprechender Diyhdrofuroimidazolderivate mit einem klassischen Hydrierkatalysator wie zum Beispiel Rhodium auf Aluminiumoxid.
Bezüglich der erreichbaren Ausbeute am gewünschten Diastereomeren konnte dieses Verfahren nicht restlos befriedigen
Es bestand folglich die Aufgabe ein verbessertes asymmetrisches Hydrierverfahren zu entwickeln, mit welchem man den genannten Schlüsselschritt der Biotinsynthese mit einer sehr guten Diastereoselektivität bei guter Ausbeute des Tetrahydrofuroimidazols durchführen kann.

Diese Aufgabe konnte gelöst werden mit dem erfindungsgemässen Verfahren nach Anspruch 1.

Die Dihydrofuroimidazole der allgemeinen Formel I können gemäss den Vorschriften des EP-PS 273 270 oder der EP-PS 270 076 hergestellt werden.

Zweckmässig werden als auf bekannte Weise abspaltbare Schutzgruppen für R₁ nachfolgende Gruppen eingesetzt:
Eine Phenyl-(C₁-C₆)-alkylgruppe, insbesondere eine Benzylgruppe, oder eine Naphthyl-(C₁-C₆)-alkylgruppe. Deren aromatische Kerne können gegebenenfalls mit einem oder mehreren Substituenten aus der Reihe (C₁-C₆)-Alkyl, (C₁-C₆)-Alkoxy, Hydroxy, einem Halogen, Amino, (C₁-C₆)-Alkylamino, (C₁-C₆)-Oialkylamine substituiert sein.
Die Phenyl-(C₁-C₆)-alkylgruppe oder die Naphthyl-(C1-C6)-alkylgruppe kann ein chirales Zentrum aufweisen.

R₁ hat bevorzugt die Bedeutung einer (R)- oder (S)-1-Phenylethylgruppe, einer Benzylgruppe oder einer (R)- oder (S)-1-Naphthylethylgruppe, wobei die aromatischen Kerne der bevorzugten Gruppen mit den genannten Substituenten substituiert sein können.

R₂ kann für Wasserstoff oder für auf bekannte Weise abspaltbare Schutzgruppen der Reihe (C₁-C₆)-Alkanoyl, Benzyl, (C₁-C₆)-Alkoxy-(C₁-C₆)-alkyl, (C₁-C₆)-Alkoxycarbonyl oder Aroyl wie z. B. Benzoyl stehen. Der aromatische Kern der Benzylgruppe oder der Aroylgruppen kann den aromatischen Kernen von R₁ entsprechend substituiert sein.

R₂ hat bevorzugt die Bedeutung von Acetyl, Benzyl, (C₁-C₂)-Alkoxy-(C₁-C₂)-alkyl, (C₁-C₂)-Alkoxycarbonyl oder Benzoyl.

Überraschenderweise wurde gefunden, dass Homogenkatalysatoren, die erhältlich sind durch Umsetzung eines Rh Komplexes mit einem chiralen Phosphin-Liganden aus der Reihe 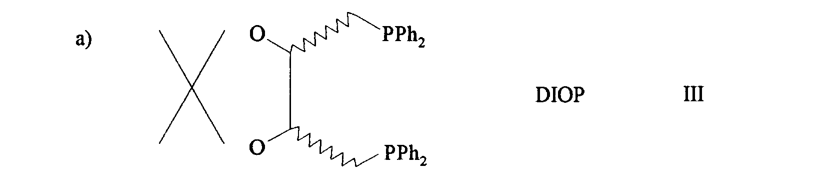 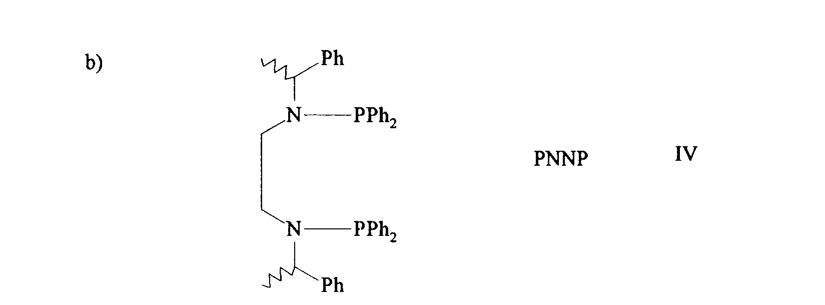 worin Ph eine Phenylgruppe bedeutet, eine, im Vergleich zum Verfahren aus dem Stand der Technik, stark erhöhte Diastereoselektivität bei gleichzeitig guter Ausbeute zeigen.

Bevorzugter DIOP-Ligand ist der (-)-DIOP-Ligand. Bevorzugter PNNP-Ligand ist der (S,S)-PNNP-Ligand. Sowohl DIOP als auch die PNNP-Liganden sind literaturbekannt. T.P: Dang et al.; J. Chem. Soc., Chem. Commun. 1971, 481 (DIOP). M. Fiorini et al.; J. Mol. Catal. 1979, 5, 303 und J. Mol. Catal. 1980, 7, 411 (PNNP).

Als Rh Komplexe finden solche der allgemeinen Formel 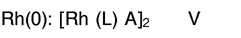 oder 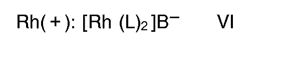 Anwendung, worin L für zwei C₂-C₁₂ Olefine oder ein C₅-C₁₂ Dien steht, A ein Halogen und B- ein Anion einer Sauerstoffsäure oder Komplexsäure bedeutet.

L in der Bedeutung als Olefin enthält bevorzugt 2 bis 6 C-Atome und in der Bedeutung als Dien bevorzugt 5 - 8 C-Atome. Das Dien kann dabei offenkettig, mono- oder bicyclisch sein. Beispiele für Olefine sind: Ethylen, Propen oder 1-Buten. Beispiele für Diene sind: 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien, Norbornadien. Bevorzugt stellt L zwei Ethylen, 1,5-Hexadien, 1,5-Cyclooctadien oder Norbornadien dar.

A steht bevorzugt für Chlor oder Brom. Beispiele für B⁻ sind: ClO₄⁻, FSO₃⁻, CH₃SO₃⁻, CF₃SO₃⁻, BF₄-, PF₆⁻, SbCl₆⁻, AsF₆⁻ und SbF₆-. Bevorzugt bedeutet B-: BF₄-, ClO₄⁻, PF₆⁻, CF₃SO₃⁻ und SbF₆-.

Die Herstellung dieser Rh Komplexe ist bekannt und geht zum Beispiel aus J. Chatt et al., J. Chem. Soc. 1957, 4735 oder aus G. Giordano et al., Inorg. Synth. 1979, 19, 218 hervor.

Die Herstellung des aktiven Homogenkatalysators erfolgt zweckmässig in situ, das heisst im Rahmen der Hydrierung des betreffenden Dihydrofuroimidazols der allgemeinen Formel I.

Zweckmässig wird so vorgegangen, dass zunächst die Homogenkatalysatorkomponenten, das heisst der Rh Komplex und der entsprechende Phosphin-Ligand, zusammen mit dem entsprechenden Dihydrofuroimidazolderivat in einem geeigneten inerten Lösungsmittel vorgelegt werden. Durch entsprechende Vorkehrungen ist zu achten, dass die Reaktion vorteilhaft in einer sauerstoffreien Inertgasatmosphäre durchgeführt wird.

Als Lösungsmittel die alleine oder im Gemisch angewendet werden können, eignen sich zweckmässig aprotische Lösungsmittel wie z. B. die aliphatischen oder aromatischen Kohlenwasserstoffe oder die halogenierten Kohlenwasserstoffe. Geeignete Vertreter der aromatischen Kohlenwasserstoffe sind z. B. Benzol, Toluol oder Xylol, der aliphatischen Kohlenwasserstoffe z. B. Hexan oder Pentan und der halogenierten Kohlenwasserstoffe Methylenchlorid oder Chloroform. Besonders geeignetes Lösungsmittel ist Toluol. Es kann unter Umständen von Vorteil sein in einer Mischung der genannten Lösungsmittel mit einem protischen Lösungsmittel wie z. B. mit aliphatischen Alkoholen zu arbeiten. Besonders geeigneter aliphatischen Alkohol ist Methanol.

Die Lösungsmittelmenge wird zweckmässig so gewählt, dass eine Substratkonzentration von 2% bis 30% erhalten wird. Bevorzugt wird bei einer Substratkonzentration von 10% gearbeitet.

Die Katalysatormenge ausgedrückt als Verhältnis Substrat (Dihydrofuroimidazol) zu Katalysator bewegt sich zweckmässig zwischen 100 : 1 und 5000 : 1 bevorzugt im Bereich um 500 : 1.

Die Umsetzung erfolgt von Vorteil bei einem Wasserstoffdruck zwischen 1 bar und 200 bar vorzugsweise 1 bar bis 20 bar und bei einer Reaktionstemperatur zwischen 25 _{°} C und 150°C vorzugsweise 40 _{°} C und 90_{°}C.

Nach der Hydrierung kann das gewünschte diastereomere (3aS-6aR)-Tetrahydrofuroimidazol der allgemeinen Formel 11 auf fachmännische Weise isoliert werden.

Durch Umkristallisation mit einem geeigneten Lösungsmittel wie zum Beispiel Methylisobutylketon, Ethylacetat oder Toluol können allfällige Anteile des unerwünschten (3aR-6aS)-Tetrahydrofuroimidazols entfernt werden.

Die resultierenden Tetrahydrofuroimidazole können dann zum Beispiel entsprechend der EP-PS 273 270 weiter in das (+) Biotin umgesetzt werden.

Die Tetrahydrofuroimidazolderivate der allgemeinen Formel II worin R₁ eine Phenyl(C₁-C₆)-alkylgruppe bedeutet, deren aromatischer Kern mit einem oder mehreren Substituenten aus der Reihe (C₁-C₆)alkyl, (Cᵢ -C₆)-Alkoxy, Hydroxy, einem Halogen, Amino, (C1-C6)-Alkylamino oder (C₁-C₆)-Oialkylamino substituiert ist oder worin R₁ eine Naphthyl-(C₁-C₆)-alkylgruppe bedeutet die gegebenenfalls mit den genannten Substituenten substituiert ist und R₂ die genannte Bedeutung hat, sind nicht literaturbekannt und damit ebenfalls Bestand-teil der Erfindung.

Bevorzugt steht bei diesen neuen Verbindungen R₁ für eine (R)- oder (S)-1-Phenylethylgruppe deren aromatischer Kern mit einem oder mehreren der genannten Substituenten substituiert ist oder für eine (R)-oder (S)-1-Naphthylethylgruppe.

### Beispiel 1

### Herstellung von (3aS,6aR)-Tetrahydro-1-[(R)-1-phenylethyl]furo[3,4-d]imidazol-2,4-dion.

Ein Autoklav wurde unter Sauerstoffausschluss mit 3.4 g 3,6-Dihydro-1-[(R)-1-phenylethyl]-1H-furo[3,4-d]imidazol-2,4-dion, 13.7 mg Chlorrhodium (1,5-cyclooctadien)-dimer und 27.9 mg (-)-DIOP versehen. Nach Spülen mit Argon wurden 75 ml sauerstoffreies Toluol zugegeben. Bei einem H₂-Druck von 14 bar und einer Temperatur von 90 _{°} C wurde die Reaktion während 4 Studen laufen gelassen. Die HPLC Analyse zeigte daraufeinen 98% Umsatz und eine Diastereoselektivität (de) von 54% bezüglich dem gewünschten RRS Isomer. Der Autoclav wurde entspannt und mit Stickstoff gespült. Das Reaktionsgemisch wurde filtriert. Das Rohprodukt wurde in Ethylacetat umkristallisiert. Man erhielt 2.25 g (65%) des reinen Titelproduktes. Schmelzpunkt: 156°C - 158°C [a]₅₄₆ ⁺253° (C ⁼ 1,0 CHCI₃)

### Beispiel 2

### Herstellung von (3aS,6aR)-Tetrahydro-1-[(R)-1-phenylethyl]furo[3,4-d]imidazol-2,4-dion.

Ein Autoklav wurde unter Sauerstoffausschluss mit 3.4 g 3,6-Dihydro-1-[(R)-1-phenylethyl]-1H-furo[3,4-d]imidazol-2,4-dion, 13.7 mg Chlorrhodium (1,5-cyclooctadien)-dimer und 27.9 mg (S,S)-PNNP versehen. Nach Spülen mit Argon wurden 75 ml sauerstoffreies Toluol zugegeben. Bei einem H₂-Druck von 14 bar und einer Temperatur von 90 _{°} C wurde die Reaktion während 4 Studen laufen gelassen. Die HPLC Analyse zeigte daraufeinen 98% Umsatz und eine Diastereoselektivität (de) von 54% bezüglich dem gewünschten RRS Isomer. Der Autoclav wurde entspannt und mit Stickstoff gespült. Das Reaktionsgemisch wurde filtriert. Das Rohprodukt wurde in Ethylacetat umkristallisiert. Man erhielt 2.3 g (67%) des reinen Titelproduktes. Schmelzpunkt: 157°C-158°C [a]₅₄₆ ⁺254° (C ⁼ 1,0 CHCl₃)

### Beispiel 3

### Herstellung von (3aS,6aR)-3-Benzyltetrahydro-1-[(R)-1-phenylethyl]furo[3,4-d]imidazol-2,4-dion

Im wesentlichen erfolgte die Durchführung gemäss Beispiel 1. Das Molverhältnis Edukt / Katalysator betrug 500/1. Die Reaktion wurde bei 70 °C und einem H₂ Druck von 50 bar während 60 h laufen gelassen. Man erhielt einen de von 50 %.

¹H-NMR (CDCl₃, 400 MHz) δ in ppm 1,57 (d, 3H, J = 7,1 Hz); 5,34 (q, 1 H) 5,03 (d, 1 H, J = 14,8 Hz); 4,32 (d, 1 H); 3,89 (d, 1 H, J = 9,2 Hz); 4,35 (m, 1 H, J = 5,7 Hz); 3,82 (dd, 1 H, J = 2,7 Hz); 3,38 (df, 1 H, J = 10,4 Hz); 7,3 - 7,4 (m, 10H).

### Beispiel 4

### Herstellung von (3aS,6aR)-Tetrahydro-1-(2-methoxybenzyl)furo[3,4d]imidazo-2,4-dion

Im wesentlichen erfolgte die Durchführung gemäss Beispiel 1. Das Molverhältnis Edukt / Katalysator betrug 250/1. Die Reaktion wurde bei 70 °C und einem H₂ Druck von 75 bar während 18 h laufen gelassen. Man erhielt einen de von 60 %.

¹H-NMR (CDCl₃, 400 MHz) δ in ppm 3,84 (s, 3H); 4,12 (d, 1 H, J = 7,7 Hz); 4,25 - 4,35 (m, 3H); 4,45 (d, 1H, J = 10,1 Hz); 4,64 (d, 1 H, FJ = 14,8 Hz); 5,63 (s, 1 H); 6,89 (d, 1 H, J = 8,1 Hz); 6,95 (t, 1 H, J = 7,5 Hz); 7,27 - 7,32 (m, 2H).

### Beispiel 5

### Herstellung von (3aS,6aR)-Tetrahydro-1-[(R)-1-(2-methoxyphenyl)ethyl]furo[3,4-d]imidazol-2,4dion

Im wesentlichen erfolgte die Durchführung gemäss Beispiel 1. Das Molverhältnis Edukt / Katalysator betrug 130/1. Die Reaktion wurde bei 70 °C und einem H₂ Druck von 85 bar während 18 h laufen gelassen. Man erhielt einen de von 50 %.

¹H-NMR (DMSO-d₆, 400 MHz) δ in ppm 1,59 (d, 3H, J = 7,5 Hz); 3,59 (d, 1 H, J = 11 Hz); 3,85 (s, 3H); 3,95 (dd, 1 H, J = 11 + 5 Hz); 4,17 (d, 1 H, J = 9 Hz); 4,53 (m, 1 H); 5,42 (s, 1 H); 5,43 (q, 1 H); 6,91 (d, 1 H, J = 8 Hz); 6,98 (t, 1 H, J = 7 Hz); 7,25 - 7,40 (m, 1 H).

### Beispiel 6

Herstellung von (3aS,6aR)-3-Acetyltetrahydro-1-[(R)-1-phenylethyl]furo[3,4-d]imidazol-2,4-dion

Im wesentlichen erfolgte die Durchführung gemäss Beispiel 1. Das Molverhältnis Edukt / Katalysator betrug 500 / 1. Die Reaktion wurde bei 70 °C und einem H₂ Druck von 30 bar während 60 h laufen gelassen. Man erhielt einen de von 33 %.

¹H-NMR (CDCl₃, 400 MHz) 0 in ppm 1,65 (d, 3H, J = 6,5 Hz); 2,55 (s, 3H); 3,57 (d, 1 H, J = 10 Hz); 4,08 (dd, 1 H, J = 4,0 Hz); 4,58 (dd, 1 H, J = 9 Hz); 5,27 (d, 1 H, J = 9 Hz); 5,30 (m, 1 H).

### Beispiel 7

Herstellung von (3aS,6aR)-1-Benzyltetrahydrofuro[3,4-d]imidazol-2,4-dion

Im wesentlichen erfolgte die Durchführung gemäss Beispiel 1. Das Molverhältnis Edukt / Katalysator betrug 500/1. Die Reaktion wurde bei 70 °C und einem H₂ Druck von 30 bar während 48 h laufen gelassen. Man erhielt einen de von 50 %.

¹H-NMR (CDCl₃, 400 MHz) δ in ppm 4,19 (d, 1 H, J = 8,0 Hz); 4,25 - 4,32 (m, 4H); 4,68 (d, 1 H, J = 15,2 Hz); 5,19 (s, 1 H); 7,24 - 7,39 (m, 5H).

### Beispiel 8

Herstellung von (3aS,6aR)-Tetrahydro-1-[(R)-1-naphtalin-1-ylethyl]furo[3,4-d]imidazol-2,4-dion

Im wesentlichen erfolgte die Durchführung gemäss Beispiel 1. Das Molverhältnis Edukt / Katalysator betrug 125 / 1. Die Reaktion wurde bei 70 °C und einem H₂ Druck von 75 bar während 18 h laufen gelassen. Man erhielt einen de von 72 %.

¹H-NMR (CDCl₃, 400 MHz) δ in ppm 1,70 (d, 3H, J = 7,5 Hz); 2,66 (d, 1 H, J = 11 Hz); 3,56 (dd, 1 H, J = 11 + 5 Hz); 4,20 (d, 1 H, J = 9 Hz); 4,53 (m, 1 H); 5,67 (s, 1 H); 6,08 (q, 1 H); 7,15 - 8,25 (m, 7H).

## Patentansprüche

1. Verfahren zur asymmetrischen Hydrierung von Dihydrofuroimidazolderivaten der allgemeinen Formel 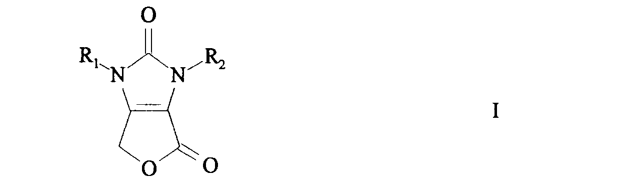 worin R₁ eine auf bekannte Weise abspaltbare Schutzgruppe und R₂ Wasserstoff oder eine auf bekannte Weise abspaltbare Schutzgruppe bedeutet mit Wasserstoff in Gegenwart eines Homogenkatalysators zu den entsprechenden diastereomeren Tetrahydrofuroimidazolderivaten der allgemeinen Formel 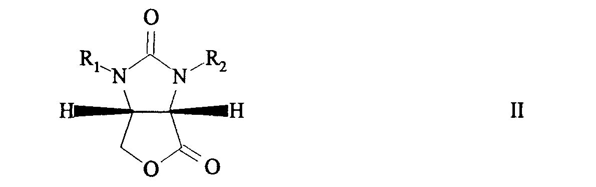 worin R₁ und R₂ die genannte Bedeutung haben, dadurch gekennzeichnet, dass Homogenkatalysatoren eingesetzt werden, die erhältlich sind durch Umsetzung eines Rh Komplexes mit einem chiralen Phosphin-Liganden aus der Reihe 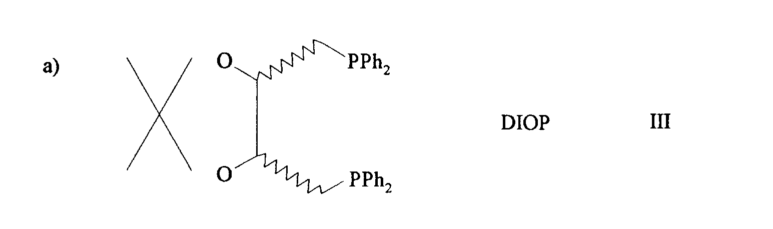 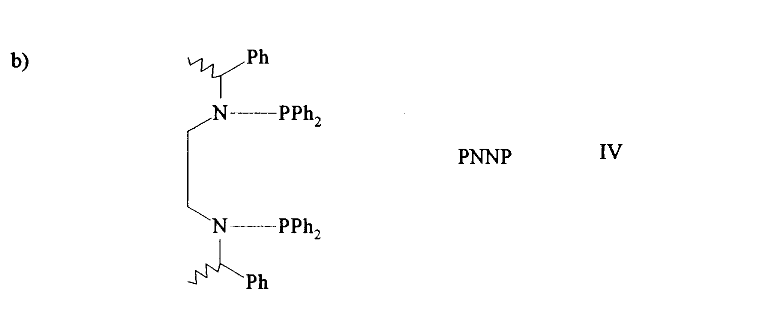 worin Ph eine Phenylgruppe bedeutet.

2. Verfahren nach Patentanspruch 1 dadurch gekennzeichnet, dass als auf eine bekannte Weise abspaltbare Gruppen für R₁ eine Phenyl-(C₁-C₆)-alkylgruppe, eine Benzylgruppe oder eine Naphthyl-(C₁-C₆)-alkylgruppe eingesetzt wird, wobei die aromatischen Kerne der jeweiligen Gruppen gegebenenfalls mit einem oder mehreren Substituenten aus der Reihe (C₁-C₆)-Alkyl, (C1-C6)-Alkoxy, Hydroxy, Halogen, Amino, (C₁-C₆)-Alkylamino oder (C₁-C₆)-Dialkylamino substituiert sind.

3. Verfahren nach Patentanspruch 1 oder 2 dadurch gekennzeichnet, dass als aufbekannte Weise abspaltbare Gruppen für R₂ eine (C₁-C₆)-Alkanoylgruppe, eine (C₁-C₆)-Alkoxy-(C₁-C₆)-alkylgruppe, eine (C₁-C₆)-Alkoxycarbonylgruppe, eine Aroylgruppe oder eine Benzylgruppe steht, wobei die aromatischen Kerne der jeweiligen Gruppen gegebenenfalls mit einem oder mehreren Substituenten aus der Reihe (C₁-C₆)-Alkyl, (C₁-C₆)-Alkoxy, Hydroxy, Halogen, Amino, (C1-C6)-Alkylamino oder (C₁-C₆)-Oialkylamino substituiert sind.

4. Verfahren nach einem oder mehreren der Patentansprüche 1 bis 3 dadurch gekennzeichnet, dass als chiraler Phosphin-Ligand der (-)-DIOP-Ligand oder der (S,S)-PNNP-Ligand eingesetzt wird.

5. Verfahren nach einem oder mehreren der Patentansprüche 1 bis 4 dadurch gekennzeichnet, dass Rh Komplexe der allgemeinen Formel 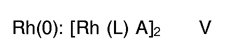 oder 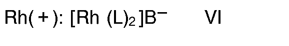 worin L fuhr zwei C₂-C₁₂ Olefine oder ein C₅-C₁₂ Dien steht, A ein Halogen und B- ein Anion einer Sauerstoffsäure oder einer Komplexsäure darstellt, eingesetzt wird.

6. Verfahren nach einem oder mehreren der Patentansprüche 1 bis 5 dadurch gekennzeichnet, dass die Umsetzung bei einem Wasserstoffdruck von 1 bis 200 bar und einer Reaktionstemperatur von 25 °C bis 150°C verläuft.

7. Verfahren nach einem oder mehreren der Patentansprüche 1 bis 6 dadurch gekennzeichnet, dass die Katalysatormenge ausgedrückt als Verhältnis Dihydrofuroimidazol zu Homogenkatalysator sich im Bereich 100 : 1 und 5000 : 1 bewegt.

8. Verfahren nach einem oder mehreren der Patentansprüche 1 bis 7 dadurch gekennzeichnet, dass aprotische Lösungsmittel eingesetzt werden.

9. Verfahren nach Patentanspruch 8 dadurch gekennzeichnet, dass Toluol eingesetzt wird.

10. (3aS-6aR)-Tetrahydrofuroimidazolderivate der allgemeinen Formel II worin R₁ eine Phenyl(C₁-C₆)-alkylgruppe bedeutet, deren aromatischer Kern mit einem oder mehreren Substituenten aus der Reihe (C₁-C₆)-Alkyl, (C₁-C₆)alkoxy, Hydroxy, einem Halogen, Amino, (C₁-C₆)-Alkylamino oder (C₁-C₆)-Dialky- lamino substituiert ist oder worin R₁ eine Naphthyl-(C₁-C₆)-alkylgruppe bedeutet, deren aromatische Kerne gegebenenfalls mit einem oder mehreren der genannten Substituenten substituiert ist.

11. (3aS,6aR)-Tetrahydrofuroimidazolderivate gemäss Patentanspruch 10 worin R1 eine (R)- oder (S)-1-Phenylethylgruppe oder eine (R)- oder (S)-1-Naphthylethylgruppe bedeutet.
